# EUROPEAN PATENT APPLICATION

(11) **EP 1 647 304 A1**
(43) Date of publication of application: **19.04.2006**
(21) Application number: 05022075.5
(22) Date of filing: 11.10.2005
(51) Int. Cl.: A61N 2/06, A61N 2/02

(54) **Magnetotherapy device**

(30) Priority: 13.10.2004 IT VI20040244
(71) Applicant: Schirato, Bruno, 36022 Cassola VI (IT)
(72) Inventor: Schirato, Bruno, 36022 Cassola VI (IT)
(74) Representative: Bettello, Pietro

(57) **Abstract**

The invention relates to a device which has a support element (1) of substantially rectangular shape composed, for example, of a mattress, inside of which there is a band magnetised in permanent manner whose two faces have opposite polarities. In the support element, an electric winding (3) is also incorporated which has one or more turns of conductor material to form a solenoid, advantageously closed on an E.M.I. absorber device of *in se* known type, normally used to reduce the line radiations of digital signals. By means of the device according to the finding, the reduction of the noise magnetic field generated by a plurality of artificial sources is obtained along with a wave emitter apparatus comparable to a magnetotherapy device, capable of producing a sequence of magnetic impulses of low frequency and of variable amplitude and frequency.

## Description

The present finding regards a device adapted to generate a beneficial action on the human body by utilising external electromagnetic fields.

It is known that the development of modern technologies and telecommunications has determined an unprecedented increase of the sources of electromagnetic fields utilised for the most diverse applications.

The strong environmental presence of these fields, which is summed to the alterations of the natural magnetic field, is held by many scholars to be one of the potential causes of alteration of cellular equilibrium and of the pathologies deriving thereof.

This has generated considerable concerns, both by scholars and ordinary people, for the possible health effects deriving from long-period exposure to electromagnetic fields (E.M.F.) present in the environment.

To begin with, it is worth making clear that all of the above is referred to non-ionising radiations, i.e. those radiations which are not able to break the molecular bonds of the cells and therefore produce mutagenic effects. Such radiations are generated by an electromagnetic field with frequency comprised, according to the scientific literature, between 0 and 10¹⁵ Hz, i.e. below the typical frequencies of ultraviolet radiation.

The device according to the finding functions in the field of the frequency of non-ionising radiations.

Various documents are present in the state of the art (for example EP-A-332086), from which a device is known composed of a mattress with variously arranged magnetic bands present inside, which have the function of mitigating the action of the external electromagnetic fields. Other documents of the state of the art, by the same applicant, include the Italian patent applications for industrial invention No. VI92A000118, VI93A000144 and VI96A000193.

These devices foresee that inside a support, which advantageously assumes the form of a covering for beds and/or couches and/or armchairs, a series of magnetic bands is present placed parallel to the inside of the aforementioned, which have the object of reducing as much as possible the effect of external electromagnetic fields.

In practice, in more or less evident manner, these devices permit obtaining around the support element a cell at whose interior the value of the magnetic field is strongly reduced.

Object of the present invention is to realise a device which, in addition to reducing the action of external electromagnetic fields is capable of generating a beneficial action on the human body.

This is obtained, according to the finding, by inserting inside a support a band-shaped structure whose two opposite faces have opposite magnetic polarities; such band is arranged essentially in "zig zag" form with a plurality of sides which substantially fill the entire width of the support element.

The device is completed by an electric winding with sides arranged parallel and in proximity with respect to the sides of said support element.

Such winding is capable of acting as a seat of induced electromotive force; finally, advantageously, the ends of said electric winding are closed on a discrete electronic component, denominated EMI absorber, also known in experts' jargon as "choke".

In practice, as will be better explained below, the device according to the finding, utilising external electromagnetic fields which are potentially dangerous for human health, permits obtaining beneficial effects on the human body and not only reducing the effect of external electromagnetic fields, as in the case of similar devices of known type.

These and other characteristics of the finding will now be described in detail below, with reference to a particular embodiment of the device according to the finding, given as a not limiting example, with the help of the attached drawing table wherein:

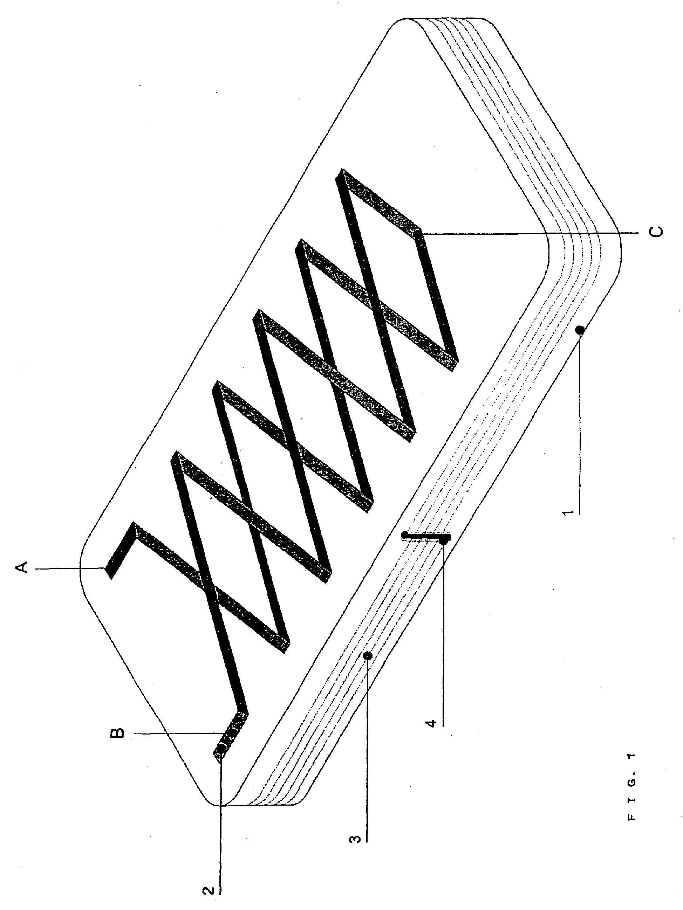

- Figure 1 represents a schematic axonometric view of the device according to the finding.

As may be seen in said figure, it is observed that the device according to the finding is composed of a support element 1 of substantially rectangular shape, composed for example of a mattress, a pillow or in general a support element of the human body or one of its parts, such that it may be utilised, for example, on a bed, couch, automobile seat, chair or simply as a rug.

Advantageously, the support element, in the case it acts as a mattress, will have a first structure having a thickness of approximately 1 cm, composed of a layer of finely sewn English Refino wool, which is then incorporated with the other elements of the finding, described below, in a single, pure cotton envelope in which the solenoid, which will be subsequently described, is locked by means of a solid border realised in polyester fibre.

Inside said support element a magnetic structure 2 is present, composed of a magnetised band whose two faces have opposite polarities. It is situated within the support element, on the side of the surface which will be placed in contact with the subject's body.

In particular, said band is arranged according to a course which develops into a "zig-zag" for the entire length of the support element. In particular, beginning with the end A, said band, after having completed the "zig-zag" course, will arrive at the end C and hence will return back, always with "zig-zag" progression, until it ends at the end B, after having covered the entire length of the support itself.

In particular it is foreseen that each time the band reaches one of the side ends of its course, in proximity to one of the side edges of the support element, the band is folded back thus to always direct a face of opposite sign, for each course section, with respect to the same reference band of the support element itself.

Advantageously, it is foreseen that, beginning from a first end A of the band-shaped structure 2 (indicatively of north polarity), once arrived at the opposite longitudinal edge of the support element 1, the structure "returns back" always with "zig-zag" progression, until it reaches the opposite longitudinal edge, where the band-shaped structure 2 will have an end section with opposite polarity with respect to that initial (indicatively, therefore, of south polarity).

In practice, at every variation of the band direction, therefore at every vertex of the aforementioned, the band is overturned so that the succession of the single sides is composed of a section with north polarity alternated with a subsequent section of south polarity and vice versa. Inside the support element 1, an electric circuit 3 is arranged with sides arranged along the perimeter of the same to form a solenoid. Such electric circuit is composed of a certain number of turns of electrically conductor material and every turn of said solenoid extends for a length roughly equal to the perimeter of the support itself and encloses an area substantially equal to the surface of the support face where the magnetic band is positioned.

Advantageously, the ends of said solenoid are closed on an EMI absorber device 4, also known in experts' jargon by the term "choke", of *in se* known type, which is composed of a discrete passive component designed to reduce the line radiations of digital signals.

Therefore, the circuit which results is completely passive, i.e. no auxiliary source of electrical energy is necessary for its operation, which implies an intrinsic safety of the device itself so to not require particular safety devices in the use of the same, as well as not require particular regulations and certifications.

The innovative functionalities implemented in the device according to the finding will now be illustrated below.

To begin with, the "zig-zag" device for the realisation of the magnetic structure produces an alternated natural magnetic field with benefits for the equilibrium restoration of human body cells.

Secondly, the electric circuit of the solenoid placed inside the support device produces an induced magnetic field composed of low frequency sequences and impulses, of variable amplitude and frequency in relation with the variations of the noise (inductor) magnetic field generated by the most varied external sources of essentially artificial type. The induced impulsive magnetic field is, therefore, utilised to produce beneficial effects on the human body, analogous to those obtainable with the application of the waves produced by emitter apparatuses (magnetotherapy apparatuses).

Indeed, the operation principle of the device according to the finding is based on the well-known Lenz's law, according to which the inductor magnetic flux linked with the solenoid varies over time, the solenoid itself becoming the seat of an induced electromotive force (e.m.f.). From the moment that the (solenoid) circuit is closed, the induced electromotive force generates a current with orientation such to generate, in turn, an induced magnetic field opposed to the inductor field.

In particular, in the case in which the E.M.I. absorber device is present, it must be considered that it is a component which has an impedance with resistive part R and inductive part X_{L} = 2 fL, (L being the self-induction coefficient of the solenoid, f being the frequency of the current induced in the solenoid). The inductive part has much greater weight with respect to the resistive part, and furthermore its value obviously depends on the frequency, which results directly proportional to the aforementioned. The solenoid-absorber set is, definitively, ascribable to a circuit, in which there is:
- a voltage generator with amplitude dependent on the number of solenoid turns, the frequency present, in addition to the speed of variation of the linked flux;
- a small resistance R in series with an inductance, X_{L}, whose value obviously depends on the frequency of the inductor field.

Clearly the current which arises in the circuit (which for convenience is assumed "with induced e.m.f. being equal") essentially varies with the X_{L} value.

Hence, the induced magnetic field, generated by this current, substantially varies in relation with the X_{L} variation, thus generating a pulsed magnetic field modulated by the inductor field.

If it is then considered that the induced e.m.f. value depends on the variations of the disturbing inductor external field, it is verified that the induced field is composed of a sequence of modulated impulses, in rather complex manner both regarding its amplitude and frequency. This produces a result similar to that produced by the most recent magnetotherapy devices.

In particular, the application of the magnetotherapy at low frequency of pulsated type has been known and codified for some time and does not require further research to confirm its effectiveness.

In summary, the original and innovative elements introduced by the physical behaviour of the device according to the finding, consist:
- in the use of the noise magnetic field, generated by various sources of artificial type and potentially dangerous for human health, not only to mitigate the potentially negative effects connected with the exposure to E.M.F. themselves, but also to obtain beneficial effects on the human body, such as those obtainable in medicine with the application of magnetotherapy:

- in the original use of the E.M.I. absorber, discrete passive component of *in se* known type, normally used to reduce the line radiations of digital signals, in order to obtain the modulated impulses as described above.

Finally, it is reaffirmed that the use of the device according to the finding is absolutely simple and effective, as its employment is safe, as well as lacks collateral effects and short-term and long-term contra-indications.

## Claims

1. DEVICE ADAPTED TO GENERATE A BENEFICIAL ACTION ON THE HUMAN BODY BY UTILISING EXTERNAL ELECTROMAGNETIC FIELDS, comprising a support element (1) of substantially rectangular shape, for example a mattress, which inside has a band-shaped structure (2), magnetised in a permanent manner, the device being **characterised in that** said structure (2) has opposite magnetic polarities at its two opposite faces, said band being arranged in "zig-zag" form with a plurality of sides which substantially fill the entire width of the support element (1), being foreseen that each time the band-shaped structure (2) reaches one of the side ends of its course, the band is folded back in order to direct a face of opposite sign at each of its transverse sections with reference to a same band of the support element (1).

2. DEVICE, according to claim 1, **characterised in that** it foresees that the structure (2) begins from one of the transverse edges of the support element (1) and therefore reaches, in "zig-zag" form, in proximity of the transverse edge opposite the first and hence returns back to the first transverse edge of departure, being foreseen that the two ends of the band-shaped structure (2) direct a face mutually opposed to the same band with reference to the support element (1).

3. DEVICE, according to claim 1 or 2, **characterised in that** in the support element (1) an electric winding (3) is also incorporated, which foresees one or more wire turns of conductor material to form a solenoid, with the sides arranged parallel and in proximity with respect to the sides of said element.

4. DEVICE, according to claim 3, **characterised in that** the winding (3) is closed at its heads on an absorber device (4) of *in se* known type, denominated E.M.I., normally utilised to reduce the line radiations of digital signals and denominated "choke" in jargon.
